# DEMANDE DE BREVET EUROPEEN

(11) **EP 1 288 185 A2**
(43) Date de publication de la demande: **05.03.2003**
(21) Numéro de dépôt: 02016690.6
(22) Date de dépôt: 26.07.2002
(51) Int. Cl.: C07C 45/86

(54) **Stabilisation de l'acroleine**

(30) Priorité: 30.08.2001 FR 0111241
(71) Demandeur: Atofina, 92800 Puteaux (FR)
(72) Inventeur: Lepizzera, Stéphane, 57500 Saint Avold (FR); Fauconet, Michel, 57730 Valmont (FR)

(57) **Abrégé**

On utilise au moins un dérivé nitroxyde pour stabiliser l'acroléine pure ou ses solutions très concentrées (teneur en acroléine ≥ 90 % en poids), en contact avec une atmosphère essentiellement anaérobie

## Description

L'invention concerne le domaine des monomères acryliques et a plus particulièrement pour objet la stabilisation de l'acroléine pour éviter son autopolymérisation lors de sa purification, son stockage ou son transport.

Le procédé de production d'acroléine le plus communément utilisé comprend une section de réaction dans laquelle est réalisée une oxydation catalytique en phase gazeuse du propylène par l'oxygène de l'air, et une section de purification pour éliminer les sous-produits de la réaction qui sont principalement les oxydes de carbone, l'acide acrylique, l'acide acétique et l'acétaldéhyde.

Un procédé de purification classique comprend une première section dite "essentiellement aqueuse" dans laquelle sont effectués successivement :
- le refroidissement du flux réactionnel aqueux (quench) ;
- l'absorption à l'eau, dans une première colonne, des acides organiques (acide acrylique et acide acétique) ;
- l'absorption à l'eau froide de l'acroléine dans une deuxième colonne avec élimination en tête des gaz incondensés (propylène non converti, azote, oxygène, oxydes de carbone) et récupération en pied de colonne d'une solution aqueuse diluée d'acroléine ;
- l'élimination de l'eau en pied d'une troisième colonne, en tête de laquelle l'acroléine est récupérée.

Pour certaines applications de l'acroléine, il s'avère souvent nécessaire de parfaire la purification dans une deuxième section de purification dite "section essentiellement organique", comprenant une quatrième colonne pour éliminer en tête des composés légers (en particulier l'acétaldéhyde) et une dernière colonne d'élimination des composés lourds.

Un des points délicats de la purification de l'acroléine provient du fait que ce monomère, de même que l'acide acrylique contenu dans les solutions brutes issues de la section réactionnelle, subit facilement une réaction d'autopolymérisation initiée par des réactions radicalaires favorisées par la température. Il s'ensuit que les flux présents dans les étapes de purification qui sont conduites à température élevée (notamment dans les colonnes de distillation) sont les plus sensibles à ce phénomène. Un deuxième facteur favorisant la polymérisation de l'acroléine est la formation de peroxydes, facilement générés par action de l'oxygène sur le monomère et connus pour initier rapidement sa polymérisation. Pour atténuer ce problème, on cherche généralement à réduire la présence d'oxygène dans le milieu, mais ceci peut avoir un effet néfaste sur l'efficacité de certains inhibiteurs de polymérisation ajoutés dans le procédé.

Les polymères générés ont la particularité d'être insolubles dans le monomère, dans les milieux bruts contenant le monomère à purifier et dans les mélanges du monomère avec les solvants utilisés dans le procédé de purification, en particulier dans les milieux aqueux. La génération de ces polymères insolubles dans les équipements industriels de distillation entraîne des bouchages qui imposent des arrêts de l'installation et des nettoyages fréquents, difficiles et coûteux.

Pour pallier ces inconvénients, il est connu d'introduire dans les flux d'acroléine une ou plusieurs molécules stabilisantes telles que des composés phénoliques (par exemple, l'hydroquinone, l'éther méthylique d'hydroquinone, le 2,6-di(t.butyl)-1-hydroxytoluène, ...), des dérivés aminés (par exemple, des hydroxylamines, l'hydroxydiphénylamine, la pipéridine, ...), des p-phénylènediamines substituées ou encore des sels de métaux de transition comme, par exemple, l'acétate de cuivre (II), l'acétate de manganèse (II) ou le dibutyldithiocarbamate de cuivre.

L'hydroquinone est souvent mentionnée comme inhibiteur dans les procédés de purification de l'acroléine ; elle ne permet cependant pas de réduire efficacement la formation de polymères durant les étapes de purification de l'acroléine. L'efficacité médiocre de cet inhibiteur et des inhibiteurs phénoliques en général s'explique par le fait que son mécanisme d'action nécessite la présence d'oxygène qui, comme indiqué plus haut, est un initiateur de la formation de peroxydes préjudiciables à la stabilité du monomère.

D'autres inhibiteurs sont plus efficaces que l'hydroquinone, mais leur efficacité est encore insuffisante pour permettre un fonctionnement sans problème de fiabilité d'une unité industrielle de purification de l'acroléine.

La présente invention vise à augmenter sensiblement la stabilité de flux riches en acroléine, en particulier lors des étapes de la section dite essentiellement organique du procédé de purification de l'acroléine. On parvient à ce résultat, sans introduction complémentaire d'oxygène ou d'un flux gazeux contenant de l'oxygène, par addition d'au moins un dérivé nitroxyde au flux d'acroléine.

L'invention a donc pour objet l'utilisation d'au moins un dérivé nitroxyde pour stabiliser l'acroléine pure ou ses solutions très concentrées (teneur en acroléine ≥ 90 % en poids), en contact avec une atmosphère essentiellement anaérobie, c'est-à-dire une atmosphère gazeuse contenant en volume moins de 1 % d'oxygène. Cette utilisation concerne plus particulièrement les étapes de la section essentiellement organique de purification de l'acroléine, mais aussi son stockage ou son transport sous atmosphère inerte.

L'invention a également pour objet un procédé de stabilisation de l'acroléine ou d'un flux riche en acroléine caractérisé en ce qu'on lui ajoute au moins un dérivé nitroxyde. La quantité de dérivé nitroxyde à ajouter peut varier dans de larges limites, mais elle est généralement comprise entre 10 et 10000 ppm par rapport au poids d'acroléine ou de flux à stabiliser, de préférence entre 50 et 5000 ppm.

Parmi les dérivés nitroxydes à utiliser selon l'invention, on peut mentionner ceux représentés par la formule générale: 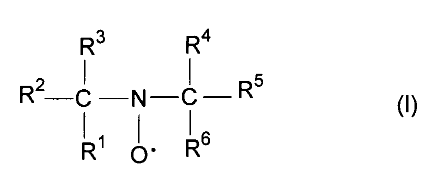 dans laquelle les symboles R¹, R², R³ et R⁴, identiques ou différents, représentent des radicaux alkyle, linéaires ou ramifiés, éventuellement substitués, les symboles R³ et R⁴ pouvant aussi être reliés entre eux; R⁵ est un atome d'hydrogène ou un radical alkyle, linéaire ou ramifié, éventuellement substitué; et R⁶ représente un radical alkyle, linéaire ou ramifié, éventuellement substitué, aryle ou dialcoxyphosphoryle. Sont plus particulièrement préférés:
- les nitroxydes cycliques de formule générale: 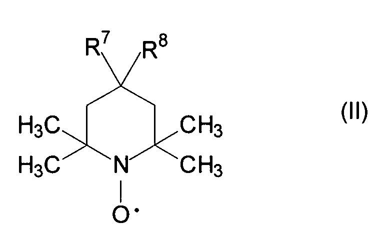 dans laquelle R⁷ représente un atome d'hydrogène ou un groupe hydroxy, acyloxy ou amino, et R⁸ représente un atome d'hydrogène ou, avec R⁷, forme un groupe oxo; et
- les nitroxydes acycliques de formule générale: 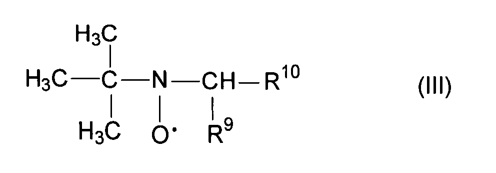 dans laquelle R⁹ représente un radical alkyle ramifié et R¹⁰ un radical phényle ou un groupe dialcoxyphosphoryle.

Comme exemples non limitatifs de ces nitroxydes préférés, on peut mentionner la 2,2,6,6-tétraméthyl-1-oxyl-pipéridine (TEMPO) et ses dérivés substitués en position 4 comme le 4-hydroxy-TEMPO, le 4-oxo-TEMPO et le 4-amino-TEMPO, ainsi que le N-tert.butyl-1-phényl-2-méthylpropyl nitroxyde et le N-tert.butyl-1-diéthylphosphono-2,2-diméthylpropyl nitroxyde (ci-après SG1) dont la formule développée et la préparation sont décrites dans la publication WO 96/24620.

Les dérivés nitroxydes selon l'invention, sous forme aqueuse ou solide, sont mélangés à l'acroléine ou au flux à stabiliser et les solutions obtenues sont ensuite injectées dans le procédé, de préférence dans le flux d'alimentation et/ou en tête de la colonne à distiller. Lorsque ces solutions sont introduites en tête de colonne, l'introduction est faite au niveau d'un ou plusieurs des points constitués par le plateau le plus élevé de la colonne ou par un plateau situé légèrement plus bas que ce dernier ou par l'entrée du condenseur des vapeurs en tête de colonne.

Les exemples suivants illustrent l'invention sans la limiter. Sauf mention contraire les parties et les pourcentages sont exprimés en poids.

### EXEMPLE 1 (Tests statiques)

Dans un tube en verre, on place de l'acroléine brute (mélange d'environ 97 % d'acroléine et 3 % d'eau) préalablement déstabilisée par distillation, puis restabilisée avec 10 ppm du stabilisant à étudier. Ce tube est surmonté d'un réfrigérant lui-même fermé par un bouchon ayant un trou pour laisser passer une canne inox. Cette canne inox sert à purger continuellement l'échantillon d'acroléine avec de l'azote. Après 20 minutes de purge à température ambiante, le tube est plongé dans un bain thermostaté à 80°C et le début de polymérisation est détecté visuellement. On définit alors pour chaque stabilisant testé une durée de stabilisation de l'acroléine dans les conditions du test.

Le tableau ci-dessous rassemble les résultats obtenus avec deux stabilisants selon l'invention (4-hydroxy-TEMPO et SG1) et, à titre comparatif, l'hydroquinone, le dibutyldithiocarbamate de cuivre (ci-après CB) et la phénothiazine.

| **Stabilisant** | **Durée de stabilisation** |
|---|---|
| 4-OH-TEMPO | > 4 h |
| SG1 | >4h |
| hydroquinone | 1 min |
| CB | 5 min |
| phénothiazine | 10 min |

### EXEMPLES 2 à 4 (Tests dynamiques)

Les tests dynamiques, destinés à comparer l'efficacité des stabilisants, ont été réalisés dans un montage permettant de simuler une colonne de distillation du procédé de purification de l'acroléine. Cette colonne, choisie parmi les plus sensibles du procédé à cause de la forte concentration en monomère dans le flux, est celle de séparation des impuretés légères, en particulier de l'acétaldéhyde.

Le montage expérimental est constitué :
- d'une colonne en verre de diamètre intérieur 36 mm, comprenant 2 sections de 14 cm de hauteur équipées chacune d'un élément de garnissage en inox de type multiknit,
- d'un bouilleur à thermosiphon en pied de colonne, chauffé par résistance électrique, et
- d'un condenseur circulé par de l'eau à 12°C en tête.

Le mélange d'alimentation est constitué d'acroléine (93 %), d'acétaldéhyde (4 %) et d'eau (3 %), dans lequel on ajoute aussi 0,1 % d'inhibiteur de polymérisation à tester. Ce mélange est introduit à un débit de 185 g/h entre les deux sections de la colonne. Le même débit de flux de pied de colonne est éliminé en continu à partir du bouilleur, de façon à maintenir un niveau constant de liquide dans le bouilleur. Le temps de séjour du flux de pied de colonne dans le bouilleur est d'une heure. La puissance de chauffe appliquée au niveau du bouilleur est adaptée de manière à obtenir un reflux liquide de débit suffisant au niveau du condenseur en tête de colonne.

Les tests sont réalisés sous pression atmosphérique. Les températures mesurées sont de 53°C au niveau du bouilleur, de 50°C au niveau de l'alimentation, et de 19°C en tête de colonne. La formation de polymère est évaluée de manière comparative, par observation des dépôts dans le montage.

### Exemple 2

L'inhibiteur de polymérisation testé est le 4-hydroxy-TEMPO, dissous à une concentration de 0,1 % en poids dans le mélange d'alimentation. Le flux de pied de colonne extrait du bouilleur est clair et limpide. Après 3 heures de fonctionnement, on n'observe aucune trace de dépôt solide dans le montage.

### Exemple 3 (comparatif)

L'inhibiteur de polymérisation testé est l'hydroquinone, dissoute dans le mélange d'alimentation à une concentration de 0,1 % en poids. Durant l'essai, on remarque que le flux liquide extrait en pied de colonne est trouble, dénotant la présence de polymères insolubles en suspension fine dans le milieu. Après 3 heures de fonctionnement, on observe un dépôt de solide blanc en couche fine tapissant la paroi interne du bouilleur sur environ la moitié de la surface, et un dépôt de solide blanc en grains dans la partie inférieure du bouilleur.

### Exemple 4 (comparatif)

Le même type d'essai est repris avec de l'éther méthylique d'hydroquinone dissous à hauteur de 0,1 % poids dans le mélange d'alimentation. Le flux de pied de colonne soutiré pendant l'opération est trouble. Après une durée d'essai de 3 heures, on observe une couche fine de solide blanc tapissant la paroi interne du bouilleur sur environ un tiers de sa surface, et un léger dépôt de solide blanc en grains dans la partie inférieur du bouilleur.

### Exemple 5

On opère comme dans les exemples 2 à 4 sauf que le mélange à tester, de même composition que dans les essais précédents, n'est pas introduit et soutiré de manière continue. C'est le flux de pied de colonne qui est renvoyé en continu, avec un débit de 200 g/h, au niveau du point d'alimentation entre les deux sections de la colonne. La durée de ce deuxième test dynamique est de 24 heures.

L'inhibiteur est le 4-OH-TEMPO, dissous dans le mélange d'alimentation à raison de 0,1 %. Après 24 heures de fonctionnement, la colonne est totalement exempte de dépôt solide et on n'observe pas de dépôt significatif sur la paroi interne du bouilleur.

### Exemple 6 (comparatif)

L'essai de l'exemple 5 est répété mais en remplaçant le 4-OH-TEMPO par l'hydroquinone ajoutée à raison de 0,1 % dans le flux d'alimentation. Après 16 heures de fonctionnement, l'essai doit être arrêté par suite du bouchage de la conduite d'alimentation. Le flux de pied de colonne contient des polymères en suspension. La paroi interne du bouilleur est recouverte d'une couche mince de solide blanc sur environ 2/3 de sa surface et on peut observer un dépôt épais de polymère visqueux au fond du bouilleur, ainsi que quelques solides en forme de grains qui en tapissent la partie inférieure.

## Revendications

1. Utilisation d'au moins un dérivé nitroxyde pour stabiliser l'acroléine dans les flux d'acroléine contenant au moins 90 % en poids de ce monomère, durant sa purification, son stockage ou son transport.

2. Utilisation selon la revendication 1, **caractérisée en ce que** le flux riche en acroléine est mis en contact avec une atmosphère gazeuse contenant en volume moins de 1 % d'oxygène.

3. Utilisation selon la revendication 1 ou 2, **caractérisée en ce que** le dérivé nitroxyde répond à la formule générale: 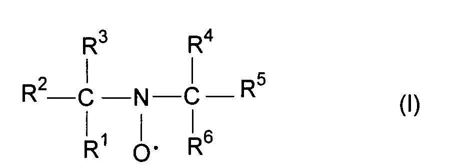 dans laquelle les symboles R¹, R², R³ et R⁴, identiques ou différents, représentent des radicaux alkyle, linéaires ou ramifiés, éventuellement substitués, les symboles R³ et R⁴ pouvant aussi être reliés entre eux; R⁵ est un atome d'hydrogène ou un radical alkyle, linéaire ou ramifié, éventuellement substitué; et R⁶ représente un radical alkyle, linéaire ou ramifié, éventuellement substitué, aryle ou dialcoxyphosphoryle.

4. Utilisation selon la revendication 1 ou 2, **caractérisée en ce que** le dérivé nitroxyde est un nitroxyde cyclique de formule générale: 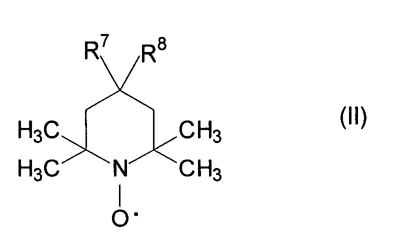 dans laquelle R⁷ représente un atome d'hydrogène ou un groupe hydroxy, acyloxy ou amino, et R⁸ représente un atome d'hydrogène ou, avec R⁷, forme un groupe oxo.

5. Utilisation selon la revendication 4 dans laquelle le dérivé nitroxyde est la 2,2,6,6-tétraméthyl-1-oxyl-pipéridine (TEMPO), le 4-hydroxy-TEMPO, le 4-oxo-TEMPO ou le 4-amino-TEMPO.

6. Utilisation selon la revendication 1 ou 2, **caractérisée en ce que** le dérivé nitroxyde est un nitroxyde acyclique de formule générale: 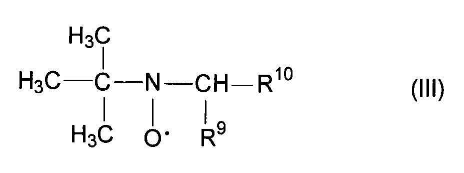 dans laquelle R⁹ représente un radical alkyle ramifié et R¹⁰ un radical phényle ou un groupe dialcoxyphosphoryle.

7. Utilisation selon la revendication 6 dans laquelle le dérivé nitroxyde est le N-tertiobutyl-1-diéthylphosphono-2,2-diméthylpropyl nitroxyde ou le N-tertiobutyl-1-phényl-2-méthylpropyl nitroxyde.

8. Utilisation selon l'une des revendications 1 à 7, **caractérisé en ce que** la concentration totale en dérivé nitroxyde dans le flux liquide d'acroléine est comprise entre 10 ppm et 10000 ppm, de préférence entre 50 et 5000 ppm, par rapport à l'acroléine.

9. Utilisation selon l'une des revendications 1 à 8, **caractérisé en ce que** le dérivé nitroxyde est utilisé pour stabiliser la section essentiellement organique d'un procédé de purification de l'acroléine.
